# EUROPEAN PATENT SPECIFICATION

(11) **EP 2 459 244 B1**
(45) Date of publication and mention of the grant of the patent: **19.03.2014**
(21) Application number: 09838030.6
(22) Date of filing: 27.07.2009
(51) Int. Cl.: A61L 27/36, A61L 27/24, A61L 27/56, A61L 27/50, A61L 27/60

(54) **METHOD FOR PREPARING POROUS COLLAGEN MATRICES**
VERFAHREN ZUR HERSTELLUNG PORÖSER KOLLAGENMATRIZEN
PROCÉDÉ POUR PRÉPARER DES MATRICES DE COLLAGÈNE POREUX

(43) Date of publication of application: 06.06.2012
(73) Proprietor: National Cheng Kung University, Tainan City 701 (TW)
(72) Inventor: HUANG, Lynn L.H., Yongkang City (TW)
(74) Representative: Chaillot, Geneviève
(86) International application number: PCT/US2009/051824
(87) International publication number: WO 2011/014154

(56) References cited:
- GB-A- 942 226
- JP-A- 2006 028 138
- US-A- 3 991 184
- US-A- 4 320 201
- US-A- 4 626 286
- US-A- 4 837 285
- US-A- 5 735 902
- US-A1- 2006 235 205

## Description

### BACKGROUND OF THE INVENTION

Collagen matrices can be used to make, among others, artificial tissues, artificial organs and drug delivery vehicles. They are typically prepared by reconstituting collagen fibers extracted from connective tissues, which are rich in collagen. Conventional methods for extracting collagen fiber require rigorous physical or chemical treatment, e.g., grinding, homogenization, or acidic/basic degradation, which destroys the network structure of collagen fibers.

US 2006/0235205 A1 discloses a method of preparing porous collagen matrices by swelling the connective tissue in an acidic solution comprising salts and afterwards removing the non-collagenous material to create a porous structure.

### SUMMARY OF THE INVENTION

This invention is based on the unexpected discovery that treating a connective tissue in a mild acidic solution substantially free of salt results in swelling of the connective tissue to a great extent without disrupting the collagen network structure.

Accordingly, one aspect of this invention features a method for preparing a porous collagen matrix directly from a connective tissue. This method includes (i) providing a connective tissue having a surface ranging from 20 mm² to 2 m² (e.g., 25 mm² to 900 cm²), (ii) swelling the connective tissue with an acidic solution by at least 50% (e.g., 100% to 500%) in volume to form a swollen connective tissue, and (iii) washing the swollen connective tissue to remove non-collagenous material, thereby forming a porous collagen matrix. The connective tissue can be derived from dermis or tendon. The acidic solution used for swelling the connective tissue has a pH of 1-6 (e.g., 2-4) and is substantially free of salt, i.e., a solution either having no salt or having salt at a very low concentration so that the ionic strength of the solution is not greater than 0.005 M. Examples of this acidic solution can be prepared from, among others, formic acid, oxalic acid, acetic acid, citric acid, lactic acid, malic acid, boric acid, phosphoric acid, or a mixture thereof. Preferably, it is a 0.1-6 M acetic acid solution.

Prior to the swelling step, the connective tissue can be rinsed with a solvent containing an organic solvent and optionally water. When the solvent contains both an organic solvent and water, the volume ratio of organic solvent:water can range from 1:4 to 9:1. Examples of the organic solvent include, but are not limited to, alcohol, ketone, acetone, acetonitrile, chloroform, N,N-dimethylformamide, dimethyl sulfoxide, and a mixture thereof. When the connective tissue is derived from skin and contains hairs or hair roots, it is preferably pre-treated with a proteolytic enzyme (e.g., dispase, trypsin, papain, pepsin, chymotrypsin, bromelain, ficin, and a mixture thereof) to remove the hairs or hair roots.

The swelling step can be performed by soaking the connective tissue in the acidic solution. Optionally, the soaking process is performed concurrently with squirting a liquid into the connective tissue or with ultrasound treatment.

After the swelling step, the resultant swollen connective tissue can be washed to remove non-collagenous material, thereby producing a porous collagen matrix. The washing step can be performed by soaking the swollen connective tissue in a wash solution containing a detergent, a proteolytic enzyme, or a mixture thereof. Optionally, this soaking process is carried out together with squirting a liquid into the swollen connective tissue or treating the tissue with ultrasound.

Also within the scope of this invention is a porous collagen matrix prepared by the method described above.

The details of one or more embodiments of the invention are set forth in the description below. Other features or advantages of the present invention will be apparent from the following drawings, detailed description of several examples, and also from the appended claims.

### BRIEF DESCRIPTION OF THE DRAWINGS

The drawings are first described.
FIG. 1 is a scanning electron microscope (SEM) image of a porous collagen matrix prepared by the process described in Example 1 below (magnification: ×100);
FIG. 2 is a SEM image of the porous collagen matrix prepared by the process described in Example 1 below (magnification: ×400).

### DETAILED DESCRIPTION OF THE INVENTION

Described herein is a method of preparing porous collagen matrices directly from a connective tissue, in which collagen is the major protein. Collagen is a triple-helix, rod-shaped molecule having a length of about 300 nm and a diameter of about 1.5 nm. A number of collagen molecules form a collagen fibril and a bundle of collagen fibrils form a collagen fiber. Covalent cross-linking exists inter- and intra-collagen molecules, thereby forming a fibrous network in a connective tissue.

In the method of this invention, the starting material, i.e., a connective tissue, can be derived from an animal, e.g., cattle, pig, horse, sheep, chicken, duck, turkey, goose, whale, and shark. The connective tissues suitable for use in this method include, but are not limited to, dermis, subcutaneous tissue, ligament, tendon, aponeurose, cartilage, and bone tissue. If necessary, a connective tissue is first cleaned manually (e.g., by gross dissection) or mechanically to remove undesirable materials such as fat and lipid. In one example, a dermis is obtained by removing lipid from a fresh animal skin, washing the skin with saline several times, and removing the surface layer of the animal skin with a dermatome to obtain the dermis. The dermis can be further washed with a phosphate buffered saline solution.

If desired, a connective tissue can be first treated with a suitable organic solvent or a mixture of the organic solvent and water to allow penetration of the organic solvent into the connective tissue. Examples of the organic solvents include, but not limited to, alcohol, ketone, acetone, acetonitrile, chloroform, N,N-dimethylformamide, dimethyl sulfoxide, or a mixture thereof. When a mixture of an organic solvent and water is used, the ratio between the organic solvent and water is above 1:5 (e.g., 1:4, 1:1, or 4:1).

When a connective tissue contains hairs or hair roots, it can be treated with a proteolytic enzyme (e.g., dispase, trypsin, papain, pepsin, chymotrypsin, bromelain, ficin, or a mixture thereof) that breaks down the hairs or hair roots.

Any of the connective tissues described above is then soaked in an effective amount of an acidic solution for a sufficient period to allow swelling of the connective tissue to a desired level, i.e., having a thickness of at least about 50% greater than (e.g., 2-10 times of) the original thickness. The acidic solution can be prepared from an organic acid, e.g., formic acid, oxalic acid, acetic acid, citric acid, lactic acid, malic acid, boric acid, phosphoric acid, or mixtures thereof. In one example, the acidic solution is an acetic acid solution having a concentration of 0.1 to 6 M (e.g., 0.1-2 M or 0.5-1.25 M). To achieve a better swelling effect, the acidic solution used in the present invention is substantially free of salt.

In the swelling step, the connective tissue is suspended in the acidic solution described above. If desired, a stream of liquid or a plurality of liquid streams can be applied to the connective tissue to facilitate penetration of the acidic solution into the tissue and reducing the time needed for swelling the connective tissue to a desired level. The liquid streams can be jetted out from a nozzle or an orifice installed in a container, where the connective tissue and the acidic solution are placed.

Alternatively or in addition, an ultrasonic wave, generated by a supersonic vibration device, or a high frequency water wave, generated by an electromagnetic field or a cam, can be applied to the connective tissue soaked in the acidic solution to help penetration of the acidic solution into the connective tissue.

The swollen connective tissue obtained from the swelling step described above is washed using a wash solution to remove substantially the non-collagenous material from the swollen connective tissue, thereby producing a porous collagen matrix. The wash solution can contain a detergent, a chelating agent, a proteolytic enzyme, or a mixture thereof.

Exemplary detergents for preparing the wash solution include, but are not limited to, sodium dodecyl sulphate (SDS), Tego compounds (e.g., Tween 80, Triton W. R. 1339, p-isooctylpolyoxy-ethylene phenol polymer, and Triton A20), cetylpyridinium chloride, cetyltrimethyl-ammonium bromide, dioctyl sodium sulphosuccinate, Emasol 4130 (polyoxyethylene sorbitan monoleate), Lubrol W, Nonidet P40. In one example, a wash solution containing 0.01 to 10% of SDS is used to treat the swollen connective tissue at 4 to 45 °C for 1 to 150 hours.

Chelating agents contained in the wash solution include, but are not limited to, ethylene diamine tetra-acetic acid (EDTA), 1,4,7,10-tetraazacyclododecane-1,4,7,10-tetraacetic acid (DOTA), 1,4,7,10-tetraazacyclododecane-1,4,7,1-0-tetrakis(methylene phosphonic acid) (DOTP), trans-1,2-diaminocyclohexant- etra-acetic acid (CDTA), 4,5-dihydroxybenzene-1,3-disulphonic acid (Tiron), thiourea, 8-hydroxyquinoline-5-sulphonic acid, 3,6-disulpho-1,8-dihydroxy- naphthalene, Eriochromeschwarz T (1-(1-hydroxy-2-naphthylazo)-2-hydroxy-5-- nitro-4-naphthalene sulphonic acid), ammonium purpurate, etc. Preferably, the chelating agent is EDTA at a concentration of 0.01 to 100 mM.

Alternatively or in addition, the wash solution can contain one or more proteolytic enzyme, e.g., ficin, pepsin, trypsin, dispase, and hermolysin, for removing extracellular matrix associated proteins, other non-collagenous proteins and telopeptide of collagen molecules. Conditions used in a limited enzyme digestion, i.e., degrading non-collagenous proteins while maintaining the integrity of collagen fibers, are well known in the art.

In the washing step, the swollen connective tissue can be suspended in any of the wash solutions mentioned above for a sufficient time. In one example, a stream of liquid or a plurality of liquid streams are jetted out from a nozzle or an orifice towards the swollen tissue to facilitate removal of non-collagenous materials. The liquid stream can be a stream of water, a detergent-containing solution, or an enzyme-containing solution. In another example, the swollen tissue, soaked in the wash solution, is treated by ultrasound to improve wash efficiency.

The porous collagen matrix obtained from the washing step can be frozen slowly to allow formation of a matrix containing liquid crystals with desired sizes. It can then be lyophilized for preservation. Alternatively, it can be soaked in a phosphate buffered saline solution and stored at 4 °C. When necessary, the porous collagen matrix can be crosslinked by standard chemical or physical methods. Agents for cross-linking collagen molecules include glutaraldehyde, formaldehyde, carbodiimides, and certain polyepoxy compounds (e.g., glycol diglycidyl ether, polyol polyglycidyl ether and dicarboxylic acid diglycidylester).

The above-described method for preparing collagen matrices differs from the conventional methods in at least two aspects. First, it does not require rigorous physical or chemical treatment (e.g., grinding, homogenization, or harsh acidic/basic treatment) that disrupts the fibrous collagen network in connective tissues. Second, it uses an acidic solution substantially free of salt to swell a connective tissue, while salt is commonly used in the conventional methods for stabilizing collagen fibers.

Also disclosed herein is a porous collagen matrix prepared by any of the methods described above. The porous collagen matrix can be used for preparing artificial tissues, artificial organs, implants, and drug delivery systems, or used as a scaffold for cell growth. Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever.

### Example 1: Preparation of Porous Collagen Matrix from Pig Skin

The skin of a pig was harvested. After removal of lipids, the skin was washed a few times with saline. The surface layer of the skin was removed with a dermatome to obtain the dermis having a thickness of 0.3 mm. The dermis was further washed with a phosphate buffered saline. After washing, any saline residue was completely removed from the surface of the dermis.

The dermis was placed in a container filled with 0.5 M acetic acid and incubated at 37°C for various periods to allow swelling of the dermis. During incubation, the container was placed on a shaker to allow suspension of the dermis. The thicknesses of the swollen dermis at different time points are shown in Table 1 below:

**Table 1. Thicknesses of Swollen Dermis at Various Time Points after Acetic Acid Treatment**

| | Acetic acid treating time | | | | |
|---|---|---|---|---|---|
| | 1.5 days | 2 days | 3 days | 4 days | 5 days |
| Thickness of Swollen Dermis | 0.45 mm (1.5x) | 0.6 mm (2x) | 0.9 mm (3x) | 1.2 mm (4x) | 2.4∼3 mm (8x∼10x) |

The swollen dermis was then soaked in a solution containing SDS (0.5%) and EDTA (0.5mM) for 2 hours at room temperature to remove non-collagenous material and produce a porous collagen matrix.

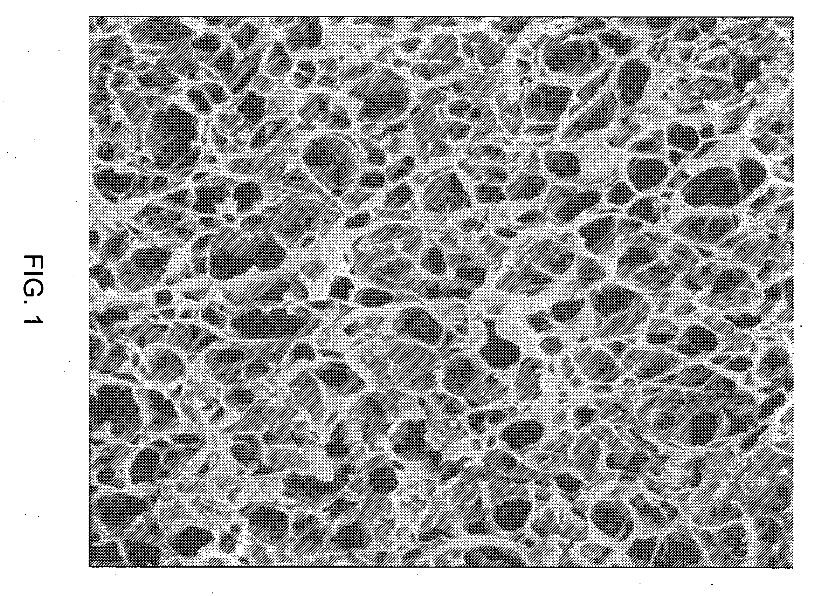

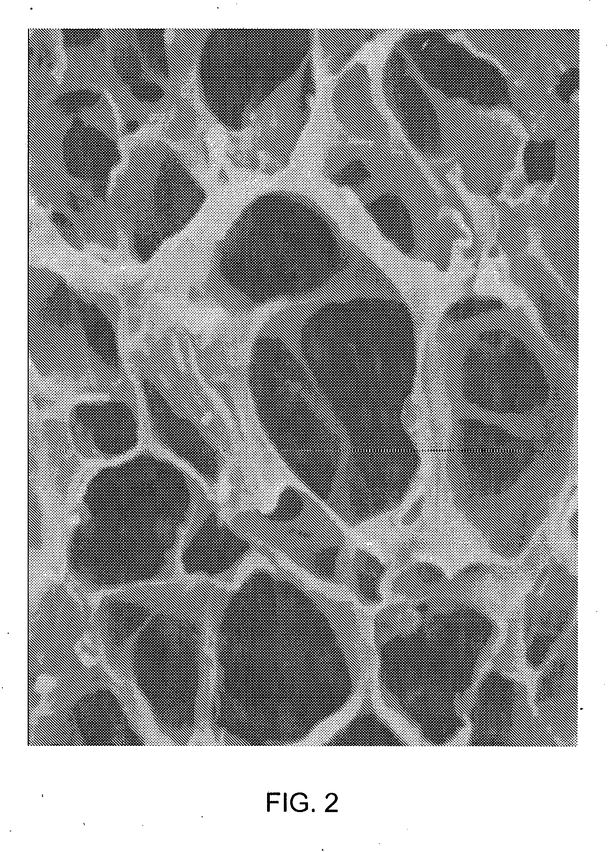

The porous collagen matrix thus obtained was washed with an aseptic phosphate buffered saline solution to remove the residual SDS and EDTA. It was then frozen at -20°C with a subsequent lyophilization for preservation. The lyophilized porous collagen matrix was sputtered with gold in vacuum and photographed under SEM. As shown in FIG. 1 and FIG. 2,
the porous collagen matrix is free of cells and cellular debris and has a matrix structure formed by collagen fibers.

### Example 2: Preparation of Porous Collagen Matrix Using Liquid Squirting

A pig dermis was prepared and soaked in 0.5 M acetic acid following the procedures described in Example 1 above. More specifically, the dermis was immersed in the acetic acid in a container having multiple nozzles, from which a number of acetic acid streams were jetted out towards the dermis. As shown in Table 2 below, this squirting step accelerated the swelling process:

**Table 2. Thicknesses of Swollen Dermis at Various Time Points after Acetic Acid Treatment**

| | Acetic acid treating time | | |
|---|---|---|---|
| | 1 day | 1.5 days | 3 days |
| Thickness of Swollen Dermis | 0.45 mm (1.5x) | 0.9 mm (3x) | 2.4∼3 mm (8x∼10x) |

The swollen dermis was then washed with the SDS/EDTA solution described in Example 1 above by both soaking and squirting to produce a porous collagen matrix.

### Example 3: Preparation of Porous Collagen Matrix Using Ultrasound Treatment

A porous collagen matrix was prepared following the same procedures described in Example 2 above except that the squirting treatment was replaced with ultrasound treatment using an ultrasonic vibration device.

Results thus obtained indicate that, like squiring, the ultrasound treatment also significantly shortened the time for swelling a dermis to a suitable level.

### Example 4: Preparation of Porous Collagen Matrix from Pig Dermis Pre-treated with Alcohol

The pig dermis prepared according to Example 1 above was placed in a container filled with 30% ethanol (alcohol/water 30:70 (v/v)). The container was placed on a shaker to allow suspension of the dermis in the ethanol solution. The dermis was incubated in the 30% ethanol solution for about 14 hrs.

After the 30% ethanol solution was decanted, the container was filled with 0.5 M acetic acid and placed on a shaker to allow swelling of the dermis soaked in the acetic acid. One day later, the swollen dermis reached a thickness of 0.45 mm (1.5 times as thick as the original dermis). This indicates that ethanol pre-treatment also shortened the swelling time to obtain a 0.45mm-thick swollen dermis.

After removing the 0.5 M acetic acid, the swollen dermis was incubated with 20% ethanol (alcohol/water 20:80 (v/v)) at room temperature for 12 hours. The porous collagen matrix thus produced was washed with deionized water and frozen at -20°C, followed by lyophilization for preservation.

### Example 5: Preparation of Porous Collagen Matrix Using Protease Treatment

A pig skin tissue was washed and then incubated in solution containing dispase (15U/ml) for one day. The skin tissue was then subjected to swelling and washing, following the procedures described in Example 2 above to produce a porous collagen matrix. Results indicate that it took only 6 hours for the dispase-treated skin tissue to reach the thickness of 0.45 mm.

### Comparative Example 1: Conventional Method for Preparing Porous Collagen Matrix

A pig dermis was obtained according to Example 1 above and soaked in 0.5 M acetic acid at 37°C for 12 hours with rotation. The treated dermis was then transferred to a solution containing 0.2 M acetic acid and 0.5 M NaCl and incubated for 72 hours. The dermis thus treated had a thickness of 0.36 mm (1.2 times as thick as the original thickness of dermis). Afterwards, the treated dermis was transferred to a solution containing 1% (w/v) hydrogen peroxide and incubated for 24 hours and subsequently transferred to a solution containing SDS and EDTA for further incubation at 37°C for 24 hours. Finally, the dermis was washed with an aseptic phosphate buffered saline solution and frozen at -20°C, followed by lyophilization.

As shown above, a 0.3-mm thick dermis reached a thickness of 0.36 mm in a > 72 hour period when swelled in a solution containing both acetic acid and salt (i.e., sodium chloride). Differently, as shown in Example 1, a 0.3-mm thick dermis reached a thickness of 0.9 mm in a three-day period (see Table 1 above), when swelled in a solution containing only acetic acid. This indicates that an acidic solution free of salt allows a dermis to swell at a much greater level than a solution containing both acid and salt (NaCl in this case). This may be due to the stabilization effect of the salt. More specifically, salt can stabilize collagen fibers so that they are closely packed, instead of swelling.

## Claims

1. A process for preparing a porous collagen matrix from a connective tissue, **characterized by** comprising:
providing a connective tissue having a surface ranging from 20 mm² to 2 m²;
swelling the connective tissue with an acidic solution by at least 50% in volume to form a swollen connective tissue, wherein the acidic solution has a pH of 1-6 and has an ion strength not greater than 0.005 M; and
washing the swollen connective tissue to remove non-collagenous material, thereby forming a porous collagen matrix.

2. The process of claim 1, wherein the connective tissue has a size of 25 mm² to 900 cm².

3. The process of claim 1, wherein the acidic solution contains an acid selected from the group consisting of formic acid, carboxylic acids, oxalic acid, acetic acid, citric acid, lactic acid, malic acid, boric acid, phosphoric acid, and a mixture thereof.

4. The process of claim 3, wherein the acidic solution has a pH value of 2 to 4.

5. The process of claim 4, wherein the acidic solution contains acetic acid at a concentration of 0.1-6 M.

6. The process of claim 1, wherein in the swelling step, the connective tissue is swollen by 100% - 500% in volume.

7. The process of claim 1, wherein the swelling step comprises soaking the connective tissue in the acidic solution and squirting concurrently a liquid into the connective tissue.

8. The process of claim 1, wherein the washing step comprises soaking the swollen connective tissue in a wash solution containing a detergent, a proteolytic enzyme, or a mixture thereof.

9. The process of claim 8, wherein the washing step comprises soaking the swollen connective tissue in the wash solution and concurrently squirting a liquid into the swollen connective tissue.

10. The process of claim 1, wherein the swelling step comprises soaking the connective tissue in the acidic solution and concurrently treating the connective tissue with ultrasound.

11. The process of claim 8, wherein the washing step comprises soaking the swollen connective tissue in the wash solution and concurrently treating the swollen connective tissue with ultrasound.

12. The process of claim 1, further comprising, prior to the swelling step, treating the connective tissue with a solvent containing an organic solvent and water, wherein the organic solvent is selected from the group consisting of alcohol, ketone, acetone, acetonitrile, chloroform, N,N-dimethylformamide, dimethyl sulfoxide, and a mixture thereof.

13. The process of claim 12, wherein the solvent contains both the organic solvent and water at a volume ratio of 1:4 to 9:1.

14. The process of claim 1, wherein the connective tissue contains a plurality of hairs or hair roots, and the process further comprises, prior to the swelling step, treating the connective tissue with a proteolytic enzyme to loosen the hairs or hair roots.

15. The process of claim 14, wherein the proteolytic enzyme is selected from the group consisting of dispase, trypsin, papain, pepsin, chymotrypsin, bromelain, ficin, and a mixture thereof.

16. The process of claim 1, wherein the connective tissue is derived from dermis or tendon.

17. The process of claim 1, further comprising, after the washing step, drying the porous collagen matrix.

18. The process of claim 1, wherein the washing step comprises treating the swollen connective tissue with a first wash solution containing a detergent, a chelating agent, or a mixture thereof, and with a second wash solution containing a proteolytic enzyme.

## Patentansprüche

1. Verfahren zur Herstellung einer porösen Kollagenmatrix aus einem Bindegewebe, **dadurch gekennzeichnet, dass** es Folgendes umfasst:
Bereitstellen eines Bindegewebes mit einer Oberfläche von 20 mm² bis 2 m²,
Quellen des Bindegewebes mit einer Säurelösung um mindestens 50% des Volumens zur Bildung eines gequollenen Bindegewebes, wobei die Säurelösung einen pH-Wert von 1-6 aufweist und eine Ionenstärke von nicht mehr als 0,005 M aufweist; und
Waschen des gequollenen Bindegewebes zum Entfernen von nichtkollagenem Material, wodurch eine poröse Kollagenmatrix entsteht.

2. Verfahren nach Anspruch 1, wobei das Bindegewebe eine Größe von 25 mm² bis 900 cm² aufweist.

3. Verfahren nach Anspruch 1, wobei die Säurelösung eine Säure enthält, die aus der Gruppe ausgewählt wird, die aus Ameisensäure, Carbonsäuren, Oxalsäure, Essigsäure, Zitronensäure, Milchsäure, Äpfelsäure, Borsäure, Phosphorsäure und einer Mischung daraus besteht.

4. Verfahren nach Anspruch 3, wobei die Säurelösung einen pH-Wert von 2 bis 4 aufweist.

5. Verfahren nach Anspruch 4, wobei die Säurelösung Essigsäure in einer Konzentration von 0,1-6 M enthält.

6. Verfahren nach Anspruch 1, wobei das Bindegewebe in dem Quellschritt um 100%-500% des Volumens gequollen wird.

7. Verfahren nach Anspruch 1, wobei der Quellschritt das Einweichen des Bindegewebes in der Säurelösung und das gleichzeitige Spritzen einer Flüssigkeit in das Bindegewebe umfasst.

8. Verfahren nach Anspruch 1, wobei der Waschschritt das Einweichen des gequollenen Bindegewebes in einer Waschlösung, die ein Detergens, ein proteolytisches Enzym oder eine Mischung daraus enthält, umfasst.

9. Verfahren nach Anspruch 8, wobei der Waschschritt das Einweichen des gequollenen Bindegewebes in der Waschlösung und das gleichzeitige Spritzen einer Flüssigkeit in das gequollene Bindegewebe umfasst.

10. Verfahren nach Anspruch 1, wobei der Quellschritt das Einweichen des Bindegewebes in der Säurelösung und das gleichzeitige Behandeln des Bindegewebes mit Ultraschall umfasst.

11. Verfahren nach Anspruch 8, wobei der Waschschritt das Einweichen des gequollenen Bindegewebes in der Waschlösung und das gleichzeitige Behandeln des gequollenen Bindegewebes mit Ultraschall umfasst.

12. Verfahren nach Anspruch 1, das ferner, vor dem Quellschritt, das Behandeln des Bindegewebes mit einem Lösungsmittel umfasst, das ein organisches Lösungsmittel und Wasser enthält, wobei das organische Lösungsmittel aus der Gruppe ausgewählt wird, die aus Alkohol, Keton, Aceton, Acetonitril, Chloroform, N,N-Dimethylformamid, Dimethylsulfoxid und einer Mischung daraus besteht.

13. Verfahren nach Anspruch 12, wobei das Lösungsmittel sowohl das organische Lösungsmittel als auch Wasser in einem Volumenverhältnis von 1:4 bis 9:1 enthält.

14. Verfahren nach Anspruch 1, wobei das Bindegewebe eine Vielzahl von Haaren oder Haarwurzeln enthält und das Verfahren ferner, vor dem Quellschritt, das Behandeln des Bindegewebes mit einem proteolytischen Enzym umfasst, um die Haare oder Haarwurzeln zu lösen.

15. Verfahren nach Anspruch 14, wobei das proteolytische Enzym aus der Gruppe ausgewählt wird, die aus Dispase, Trypsin, Papain, Pepsin, Chymotrypsin, Bromelain, Ficin und einer Mischung daraus besteht.

16. Verfahren nach Anspruch 1, wobei das Bindegewebe aus der Dermis oder Sehnen gewonnen wird.

17. Verfahren nach Anspruch 1, das ferner, nach dem Waschschritt, das Trocknen der porösen Kollagenmatrix umfasst.

18. Verfahren nach Anspruch 1, wobei der Waschschritt das Behandeln des gequollenen Bindegewebes mit einer ersten Waschlösung, die ein Detergens, ein chelatisierendes Mittel oder eine Mischung daraus enthält, und mit einer zweiten Waschlösung umfasst, die ein proteolytisches Enzym enthält.

## Revendications

1. Procédé de préparation d'une matrice de collagène poreuse à partir d'un tissu conjonctif, **caractérisé en ce qu'**il comprend :
- prendre un tissu conjonctif ayant une surface se situant dans la plage de 20 mm² à 2 m² ;
- faire gonfler le tissu conjonctif avec une solution acide d'au moins 50 % en volume pour former un tissu conjonctif gonflé, la solution acide ayant un pH de 1-6 et ayant une force ionique non supérieure à 0,005 M ; et
- laver le tissu conjonctif gonflé pour éliminer de la matière non collagénique, permettant ainsi de former une matrice de collagène poreuse.

2. Procédé selon la revendication 1, dans lequel le tissu conjonctif a une dimension de 25 mm² à 900 cm².

3. Procédé selon la revendication 1, dans lequel la solution acide contient un acide choisi dans le groupe consistant en l'acide formique, les acides carboxyliques, l'acide oxalique, l'acide acétique, l'acide citrique, l'acide lactique, l'acide malique, l'acide borique, l'acide phosphorique et un mélange de ceux-ci.

4. Procédé selon la revendication 3, dans lequel la solution acide a une valeur de pH de 2 à 4.

5. Procédé selon la revendication 4, dans lequel la solution acide contient de l'acide acétique à une concentration de 0,1-6 M.

6. Procédé selon la revendication 1, dans lequel, dans l'étape de gonflement, le tissu conjonctif est gonflé de 100 % - 500 % en volume.

7. Procédé selon la revendication 1, dans lequel l'étape de gonflement comprend le trempage du tissu conjonctif dans la solution acide et simultanément la projection d'un liquide dans le tissu conjonctif.

8. Procédé selon la revendication 1, dans lequel l'étape de lavage comprend le trempage du tissu conjonctif gonflé dans une solution de lavage contenant un détergent, une enzyme protéolytique ou un mélange de ceux-ci.

9. Procédé selon la revendication 8, dans lequel l'étape de lavage comprend le trempage du tissu conjonctif gonflé dans la solution de lavage et simultanément la projection d'un liquide dans le tissu conjonctif gonflé.

10. Procédé selon la revendication 1, dans lequel l'étape de gonflement comprend le trempage du tissu conjonctif dans la solution acide et simultanément le traitement du tissu conjonctif par des ultrasons.

11. Procédé selon la revendication 8, dans lequel l'étape de lavage comprend le trempage du tissu conjonctif gonflé dans la solution de lavage et simultanément le traitement du tissu conjonctif gonflé par des ultrasons.

12. Procédé selon la revendication 1, comprenant de plus, avant l'étape de gonflement, le traitement du tissu conjonctif par un solvant contenant un solvant organique et de l'eau, le solvant organique étant choisi dans le groupe consistant en un alcool, une cétone, l'acétone, l'acétonitrile, le chloroforme, le N,N-diméthylformamide, le diméthyl sulfoxyde et un mélange de ceux-ci.

13. Procédé selon la revendication 12, dans lequel le solvant contient à la fois le solvant organique et l'eau à un rapport en volume de 1:4 à 9:1.

14. Procédé selon la revendication 1, dans lequel le tissu conjonctif contient une pluralité de poils ou de racines de poils, et le procédé comprend de plus, avant l'étape de gonflement, le traitement du tissu conjonctif par une enzyme protéolytique pour assouplir les poils ou les racines de poils.

15. Procédé selon la revendication 14, dans lequel l'enzyme protéolytique est choisie dans le groupe consistant en la dispase, la trypsine, la papaïne, la pepsine, la chymotrypsine, la bromélaïne, la ficine et un mélange de celles-ci.

16. Procédé selon la revendication 1, dans lequel le tissu conjonctif est issu du derme ou d'un tendon.

17. Procédé selon la revendication 1, comprenant de plus, après l'étape de lavage, le séchage de la matrice de collagène poreuse.

18. Procédé selon la revendication 1, dans lequel l'étape de lavage comprend le traitement du tissu conjonctif gonflé par une première solution de lavage contenant un détergent, un agent chélatant ou un mélange de ceux-ci, et par une seconde solution de lavage contenant une enzyme protéolytique.
